# EUROPEAN PATENT APPLICATION

(11) **EP 2 957 907 A1**
(43) Date of publication of application: **23.12.2015**
(21) Application number: 14751191.9
(22) Date of filing: 21.01.2014
(51) Int. Cl.: G01N 33/48, C07K 16/28, C12Q 1/48, G01N 27/62, G01N 33/574, G01N 33/68

(54) **METHOD FOR DETECTING c-MET GENE-AMPLIFIED CELL**

(30) Priority: 15.02.2013 JP 2013027404
(71) Applicant: Fujirebio Inc., Shinjuku-ku Tokyo 163-0410 (JP)
(72) Inventor: ISE, Nobuyuki, Tokyo 163-0410 (JP); OMI, Kazuya, Tokyo 163-0410 (JP); HIGASHIYAMA, Shigeki, Toon-shi Ehime 791-0295 (JP); TAKEMORI, Nobuaki, Toon-shi Ehime 791-0295 (JP)
(74) Representative: Hoffmann Eitle
(86) International application number: PCT/JP2014/051088
(87) International publication number: WO 2014/125879

(57) **Abstract**

The present invention provides a method for detecting a c-MET gene amplification cell. Specifically, the present invention provides a method for detecting a c-MET gene amplification cell, comprising measuring a soluble c-MET in a sample utilizing a region specific for the soluble c-MET, wherein the soluble c-MET is formed by cleavage between the lysine residue (K) and the valine residue (V) in the partial region GKVI (SEQ ID NO:2) in a c-MET extracellular domain.

## Description

### TECHNICAL FIELD

The present invention relates to a method for detecting a c-MET gene amplification cell, and the like.

### BACKGROUND ART

It is shown that growth of a certain cancer can be inhibited by inhibiting a receptor-type tyrosine kinase c-MET that is one of oncogenes, and development of a c-MET inhibitor as an anticancer agent has been advanced. Although there are many unclear points so far about the cancers for which an inhibitor against the c-MET is effective, the cancers associated with abnormal activation of a c-MET (e.g., amplification of the c-MET gene, and mutation of the c-MET gene) are focused as promising candidates. Therefore, it is conceivable that diagnosis of nature of cancer attributes enables selection of the cancers to be treated with the c-MET inhibitor.

For cancers associated with amplification of the c-MET gene among the cancers associated with the abnormal activation of c-MET, a FISH method which can directly detecting the gene amplification is promising, but the FISH method is problematic for automation since it requires cumbersome steps in treatments of cancer cells and detection of the gene amplification. Thus, for the cancers associated with the amplification of the c-MET gene, it is expected to develop a more convenient diagnosis method.

As a phenomenon associated with the c-MET gene amplification, a phenomenon in which c-MET is cleaved in its extracellular domain, thereby soluble c-MET is extracellularly secreted and further migrates in blood was found (Patent Literature 1). However, the presence of a soluble splicing variant consisting of a c-MET extracellular domain-constituting polypeptide alone (Non-patent Literature 1) and a phenomenon in which c-MET is cleaved with a different protease in its extracellular domain (Non-patent Literatures 2 and 3) were reported. Thus, it is thought that cells not associated with the c-MET gene amplification also secret the soluble splicing variant and/or different soluble c-MET into blood. Therefore, in order to diagnose the cancer associated with the c-MET gene amplification with a high degree of accuracy, it is thought that it is necessary to distinctively measure a specific soluble c-MET that is to be secreted from cancer cells associated with the c-MET gene amplification from a different soluble c-MET and a soluble splicing variant that are to be secreted from cancer cells not associated with the c-MET gene amplification.

### PRIOR ART LITERATURE

### PATENT LITERATURE

Patent Literature 1: International Publication WO2011/125458

### NON-PATENT LITERATURE

Non-patent Literature 1: Jin P. et al., Arthritis Res. Ther., 2008; 10(4): R73 (Available online http://arthritis-research.com/content/10/4/R73)
Non-patent Literature 2: Kopitz C. et al., Cancer Res., 2007; 67: 8615-8623
Non-patent Literature 3: Schelter F. et al., J Biol. Chem., 2010; 285(34): 26335-40

### DISCLOSURE OF INVENTION

### PROBLEM TO BE SOLVED BY THE INVENTION

It is an object of the present invention to provide a method for detecting a c-MET gene amplification cell.

### MEANS FOR SOLVING PROBLEM

As a result of an extensive study, the present inventors have found that a specific soluble c-MET fragment specifically secreted by a c-MET gene amplification cell is formed by cleavage between the lysine residue (K) and the valine residue (V) in the partial region GKVI (SEQ ID NO:2) in a c-MET extracellular domain, this cleavage is predominantly caused by ADAM17, the c-MET gene amplification cell can be specifically detected by specifically measuring the specific soluble c-MET fragment in a sample, and the like, and completed the present invention.

Accordingly, the present invention is as follows:
[1] A method for detecting a c-MET gene amplification cell, comprising measuring a soluble c-MET in a sample utilizing a region specific for the soluble c-MET, wherein the soluble c-MET is formed by cleavage between the lysine residue (K) and the valine residue (V) in the partial region GKVI (SEQ ID NO:2) in a c-MET extracellular domain.
[2] The method of [1], which is a method of examining a cancer.
[3] The method of [1] or [2], wherein the sample is derived from a human.
[4] The method of any of [1] to [3], wherein the sample is a liquid sample.
[5] The method of any of [1] to [4], wherein the c-MET gene amplification cell is a lung cancer cell.
[6] The method of any of [1] to [5], comprising:
   (1) treating the sample with a protease; and
   (2) measuring a fragment of the soluble c-MET which has the dipeptide unit GK at its C-terminus.
[7] The method of any of [1] to [5], wherein the measurement of the soluble c-MET is carried out by detecting the fragment of the soluble c-MET which has the dipeptide unit GK at its C-terminus by mass spectrometry.
[8] The method of [6] or [7], wherein the soluble c-MET fragment is a peptide consisting of an amino acid sequence of QAISSTVLGK (SEQ ID NO:10) or KQAISSTVLGK (SEQ ID NO:11).
[9] An affinity substance for a region specific for a soluble c-MET formed by cleavage between the lysine residue (K) and the valine residue (V) in the partial region GKVI (SEQ ID NO:2) in a c-MET extracellular domain.
[10] The affinity substance of [9], wherein the affinity substance is an antibody.
[11] A diagnostic reagent for cancers, comprising an affinity substance for a region specific for a soluble c-MET formed by cleavage between the lysine residue (K) and the valine residue (V) in the partial region GKVI (SEQ ID NO:2) in a c-MET extracellular domain.

### EFFECT OF THE INVENTION

The method of the present invention is useful for examining the cancers associated with the c-MET gene amplification since it can specifically detect the c-MET gene amplification cell. The method of the present invention can also conveniently determine a therapeutic effect of an anticancer agent (e.g., molecular-targeted drugs such as a c-MET inhibitor and an EGFR inhibitor) on cancers, and can conveniently monitor the therapeutic effect of the anticancer agent on the cancers.

### BRIEF DESCRIPTION OF DRAWINGS

FIG. 1 is a view showing inhibition of c-MET secretion by knockdown of an ADAM17 gene. si-N.C.: negative control si-RNA; si-AD10: si-RNA targeting an ADAM10 gene; siAD17: si-RNA targeting the ADAM17 gene;
FIG. 2 is view showing detection of a c-MET extracellular domain fragment in culture supernatant; and
FIG. 3 is a view showing detection of a peptide fragment having 922K at its C-terminus, which is formed by digestion with trypsin, Lys-C and chymotrypsin.

### EMBODIMENT FOR CARRYING OUT THE INVENTION

The present invention provides a method for detecting a c-MET gene amplification cell.

The method of the present invention comprises measuring a soluble c-MET in a sample utilizing a region specific for the soluble c-MET, wherein the soluble c-MET is formed by cleavage between the lysine residue (K) and the valine residue (V) in the partial region GKVI (SEQ ID NO:2) in a c-MET extracellular domain.

c-MET is a receptor of a hepatocyte growth factor (HGF) and activates an intracellular signaling by binding with the HGF. c-MET is composed of the α chain and β chain generated by cleavage of a precursor of c-MET (pro c-MET) and forms a dimer by a disulfide linkage. c-MET is a receptor penetrating a cell membrane and the entire α chain and a part of the β chain are present extracellularly (see e.g., Mark et al., The Journal of Biological Chemistry, 1992, Vol. 267, No.36, pp. 26166-26171; Journal of Clinical and Experimental Medicine, 2008, Vol. 224, No.1, pp. 51-55). See e.g., GenBank accession number: NP_000236.2 (SEQ ID NO:1) for human c-MET and its α chain and β chain.

As used herein, the term "soluble c-MET" refers to a polypeptide of a partial region in the c-MET extracellular domain, which is secreted by ectodomain shedding. A cancer cell associated with the c-MET gene amplification forms a specific soluble c-MET as demonstrated in Example 1. In human, such a specific soluble c-MET consists of the partial amino acid sequence consisting of the amino acid residues at positions 1 to 922 in the amino acid sequence of SEQ ID NO:1.

Such a specific soluble c-MET is formed by the cleavage between the lysine residue (K) and the valine residue (V) in the partial region GKVI (SEQ ID NO:2) in the c-MET extracellular domain. In c-MET derived from the human, the lysine residue (K) corresponds to the amino acid residue at position 922 in the amino acid sequence of SEQ ID NO:1, and the valine residue (V) corresponds to the amino acid residue at position 923 in the amino acid sequence of SEQ ID NO:1. In the c-MET extracellular domain, the partial region GKVI (SEQ ID NO:2) to be cleaved between the lysine residue (K) and the valine residue (V) is preferably LGKVIV (SEQ ID NO:3), more preferably VLGKVIVQ (SEQ ID NO:4) and still more preferably TVLGKVIVQP (SEQ ID NO:5). The above cleavage can predominantly be caused, for example, by ADAM17 belonging to ADAM (a disintegrin and metalloprotease) family that is a one-transmembrane multi-domain protein. ADAM17 may also be called as TACE (TNF-alpha-converting enzyme).

The specific soluble c-MET formed by the above cleavage has the dipeptide unit GK at its C-terminus. The dipeptide unit possessed by such a specific soluble c-MET at its C-terminus is preferably LGK, more preferably VLGK (SEQ ID NO:6) and still more preferably TVLGK (SEQ ID NO:7).

The term "soluble (c-MET) splicing variant" refers to a polypeptide consisting of an amino acid sequence that constitutes the c-MET extracellular domain, which is formed by alternative splicing rather than ectodomain shedding. Examples of the soluble splicing variant may include variant 1 (ACF47603.1), variant 2 (ACF47604.1), variant 3 (ACF47605.1), variant 4 (ACF47606.1), variant 5 (ACF47607.1), variant 6 (ACF47608.1), variant 7 (ACF47609.1), variant 8 (ACF47610.1), variant 9 (ACF47611.1), variant 10 (ACF47612.1), variant 11 (ACF47613.1), variant 12 (ACF47614.1), variant 13 (ACF47615.1), variant 14 (ACF47616.1), and variant 15 (ACF47617.1) (see Non-patent Literature 1).

A region specific for the specific soluble c-MET refers to a region that is present in the aforementioned specific soluble c-MET but cannot be present in the soluble splicing variant. In the human, the aforementioned specific soluble c-MET consists of the partial amino acid sequence consisting of the amino acid residues at position 1 to 922 in the amino acid sequence of SEQ ID NO:1 encoding c-MET. On the other hand, any of the aforementioned soluble splicing variants has the partial region consisting of the amino acid residues at positions 1 to 861 in the amino acid sequence of SEQ ID NO:1. However, all of the soluble splicing variants cannot have the partial region consisting of the amino acid residues at positions 862 to 922 in the amino acid sequence of SEQ ID NO:1. Thus, in the human, the region specific for the specific soluble c-MET can be the partial region consisting of the amino acid residues at positions 862 to 922 in the amino acid sequence of SEQ ID NO:1.

The measurement of the specific soluble c-MET utilizing the region specific for the specific soluble c-MET can be carried out, for example, using an affinity substance for the region specific for the specific soluble c-MET. Examples of such an affinity substance may include antibodies and aptamers. The affinity substance for the region specific for the specific soluble c-MET may be, for example, an affinity substance for a peptide consisting of an amino acid sequence of QAISSTVLGK (SEQ ID NO:10) or KQAISSTVLGK (SEQ ID NO:11). The measurement of the specific soluble c-MET using such an affinity substance may also be carried out by an immunological technique. Examples of the immunological technique may include an enzyme immunoassay (EIA) (e.g., direct competitive ELISA, indirect competitive ELISA, sandwich ELISA), a radioimmunoassay (RIA), a fluorescence immunoassay (FIA), an immunochromatographic method, a luminescence immunoassay, a spin immunoassay, a western blotting method, and a latex aggregation method.

An antibody against the region specific for the specific soluble c-MET may be either a polyclonal antibody or a monoclonal antibody. The antibody may also be fragments (e.g., Fab, F(ab')₂) of the antibody, or a recombinant antibody (e.g., scFv). The antibody may be provided in a form of being immobilized on a substrate such as a plate or a form of being immersed in a support such as a strip. The antibody can be produced by a known method *per se* using a peptide consisting of the region specific for the specific soluble c-MET or a partial peptide thereof as an antigen.

The polyclonal antibody can be obtained, for example, by administering the peptide consisting of the region specific for the specific soluble c-MET or the partial peptide thereof as the antigen together with a commercially available adjuvant (e.g., complete or incomplete Freund's adjuvant) subcutaneously or intraperitoneally to an animal approximately two to four times every two or three weeks, collecting whole blood about 3 to 10 days after the final immunization, and purifying antiserum. Examples of the animal administered with the antigen may include mammals such as rats, mice, rabbits, goats, cattle, guinea pigs, and hamsters.

The monoclonal antibody can be produced, for example, by a cell fusion method. For example, a mouse is administered with the peptide consisting of the region specific for the specific soluble c-MET or the partial peptide thereof together with the commercially available adjuvant subcutaneously or intraperitoneally two to four times, spleen or lymph node is taken from the mouse about 3 days after the final immunization, and leukocytes are collected. A cell fusion of this leukocyte and a myeloma cell (e.g., NS-1) is carried out to obtain a hybridoma that produces the monoclonal antibody against the factor. The cell fusion methods may include a PEG method and a voltage pulse method. The hybridoma that produces a desired monoclonal antibody can be selected by detecting an antibody specifically binding to the antigen in a culture supernatant using a well-known EIA or RIA method or the like. The hybridoma that produced the monoclonal antibody can be cultured *in vitro,* or *in vivo* such as in ascites of a mouse or a rat, preferably the mouse, and the antibody can be obtained from the culture supernatant of the hybridoma or from the ascites of the animal.

The aptamer against the region specific for the specific soluble c-MET can be produced by a technique called SELEX.

The affinity substance for the region specific for the specific soluble c-MET may be optionally provided in a form of being labeled with a labeling substance. Examples of the labeling substance may include fluorescent substances such as FITC and FAM, luminescent substances such as luminol, luciferin and lucigenin, radioisotopes such as ³H, ¹⁴C, ³²P, ³⁵S, and ¹²³I, and affinity substances such as biotin and streptoavidin.

The measurement of the specific soluble c-MET utilizing the region specific for the specific soluble c-MET may be carried out by combining an affinity substance for a c-MET fragment having the dipeptide unit VI at its N-terminus, which is formed by the cleavage between the lysine residue (K) and the valine residue (V) in the partial region GKVI (SEQ ID NO:2) in the c-MET extracellular domain, with an affinity substance for the region specific for the specific soluble c-MET. Such a combination can measure more specifically the specific soluble c-MET having the dipeptide unit GK at its C-terminus.

The measurement of the specific soluble c-MET utilizing the region specific for the specific soluble c-MET may also be carried out, for example, by treating a sample with a protease, and subsequently detecting a peptide derived from the region specific for the specific soluble c-MET. Examples of the protease may include trypsin, chymotrypsin, Lys-C, Asp-N, Glu-C, Arg-C, asparaginyl endopeptidase, arginyl endopeptidase, and V8 protease. Such a peptide is any peptide derived from the partial region consisting of the amino acid residues at positions 862 to 922 in the amino acid sequence of SEQ ID NO:1, and examples thereof may include a peptide consisting of an amino acid sequence of QAISSTVLGK (SEQ ID NO:10) or KQAISSTVLGK (SEQ ID NO:11). The detection can be carried out, for example, by a method such as HPLC or mass spectrometry.

The measurement of the specific soluble c-MET utilizing the region specific for the specific soluble c-MET may also be carried out, for example, by mass spectrometry. In the measurement by the mass spectrometry, first a sample containing the specific soluble c-MET is directly introduced into a mass spectrometer, and subsequently bombarded with gas such as argon or nitrogen to carry out collision-induced dissociation. Then, the peptide derived from the region specific for the specific soluble c-MET (e.g., a fragment of the soluble c-MET which has the dipeptide unit GK at its C-terminus) is detected in resulting fragment ions, thereby the specific soluble c-MET can be measured.

The method of the present invention is useful for examination of cancers, for example, diagnosis of cancers associated with the c-MET gene amplification. As used herein, the term "cancer" refers to a cancer that may be associated with the amplification of the c-MET gene. Examples of such a cancer may include lung cancer (e.g., non-small cell carcinoma such as squamous cell carcinoma, adenocarcinoma and large cell carcinoma, and small cell carcinoma), gastrointestinal cancer (e.g., gastric cancer, small intestine cancer, large intestine cancer, rectal cancer), pancreatic cancer, renal cancer, liver cancer, thymic cancer, spleen cancer, thyroid cancer, adrenal cancer, prostate cancer, urinary bladder cancer, ovarian cancer, uterus cancer (e.g., endometrial cancer, cervical cancer), bone cancer, skin cancer, brain tumor, sarcoma, melanoma, blastoma (e.g., neuroblastoma), adenocarcinoma, squamous cell carcinoma, solid cancer, epithelial cancer, and mesothelioma.

The sample is not particularly limited as long as the sample likely contains the specific soluble c-MET as aforementioned, and examples thereof may include liquid samples (e.g., blood, serum, urine, saliva, ascites, a tissue extract and a cell extract) and non-liquid samples (e.g., tissue samples, cell samples). Among those mentioned above, the blood, serum, urine and saliva are preferred in terms of low invasiveness. Optionally, the sample may be previously treated before the measurement. Examples of such a treatment may include centrifugation, extraction, concentration, fractionation, cell fixation, tissue fixation, tissue freezing, and tissue sectioning.

The sample is preferably the liquid sample. When the liquid sample likely contains a cell expressing full-length c-MET (in other words, membrane-bound c-MET), the method of the present invention may comprise removing such a cell. The cell can be removed, for example, by pretreatment as aforementioned (e.g., centrifugation, fractionation). The liquid sample after removing the cell can contain the specific soluble c-MET as well as different soluble c-MET and the soluble splicing variant, but does not contain the full-length c-MET (i.e., membrane-bound c-MET having the region specific for the specific soluble c-MET as aforementioned). Thus, by utilizing the region specific for the specific soluble c-MET, it is possible to specifically measure the specific soluble c-MET.

Examples of a subject from whom the sample is derived may include a mammal that is suffered from the cancer or a mammal that is suspected of being suffered from the cancer. Examples of the mammal may include primates, pet animals, and working animals. Specifically, examples of the mammal may include humans, chimpanzees, dogs, cats, cattle, sheep, horses, pigs, rabbits, mice, and rats, and the mammal is preferably the human.

A subject having the cancer associated with the c-MET gene amplification is thought to abundantly express the substrate protein c-MET which is to be cleaved by a specific protease (e.g., ADAM17) due to the c-MET gene amplification. In such a subject, it is thought that correlating with abundantly expressed c-MET, a concentration of the specific soluble c-MET formed by cleavage of c-MET is also increased. This time, it has been found that the specific soluble c-MET is secreted by the cleavage between the lysine residue (K) and the valine residue (V) in the partial region GKVI (SEQ ID NO:2) in the extracellular domain. In order to diagnose the cancer associated with the c-MET gene amplification with a high degree of accuracy, it is necessary to distinctively measure the specific soluble c-MET which can be secreted from the cancer cell associated with the c-MET gene amplification from different soluble c-MET and the soluble splicing variant which can be secreted from the cell not associated with the c-MET gene amplification. It is thought that the aforementioned concentration of the specific soluble c-MET can be utilized as an indicator for the presence or absence and an amount of the c-MET gene amplification cell and further an indicator for determining with a high degree of accuracy whether being suffered from the cancer associated with c-MET gene amplification or not.

The subject from whom the sample is derived may be a patient with cancer for whom the treatment with a specific anticancer agent is considered. Examples of the specific anticancer agent may include a c-MET inhibitor and anticancer agents other than the c-MET inhibitor. Examples of the c-MET inhibitor may include PHA-665752, SU11274, XL-880, XL-184, ARQ 197, AMG208, AMG458, CE-355621, and MP470. Examples of the anticancer agents other than the c-MET inhibitor may include an EGFR inhibitor, an ALK inhibitor, a PDGFR inhibitor, and a c-KIT inhibitor. Example of the EGFR inhibitor may include gefitinib, erlotinib, cetuximab, lapatinib, ZD6474, CL-387785, HKI-272, XL647, PD153035, CI-1033, AEE788, BIBW-2992, EKB-569, and PF-299804. Examples of the ALK inhibitor may include WHI-P154, TAE684, and PF-2341066. Examples of the PDGFR inhibitor may include Gleevec, Desatinib, Valatinib, and Pazopanib. Examples of the c-KIT inhibitor may include Imatinib, Sunitinib, Valatinib, Desatinib, Masitinib, Motesanib, and Pazopanib. The method of the present invention is useful for selecting patients having cancers associated with the c-MET gene amplification that are sensitive to the c-MET inhibitor or resistant to the anticancer agent other than c-MET and patients having cancers not associated with the c-MET gene amplification that are resistant to the c-MET inhibitor or sensitive to the anticancer agent other than c-MET. For example, see International Publication WO2011/125458 that discloses that the cancer sensitive to the c-MET inhibitor, which can be associated with the c-MET gene amplification and is sensitive to the c-MET inhibitor can be specifically determined by measuring the concentration of the c-MET extracellular domain fragment (specific soluble c-MET).

The subject from whom the sample is derived may be a patient with cancer for whom a therapeutic effect of the specific anticancer agent is monitored. The patient with cancer may also be a patient who is required to evaluate whether a treatment with the specific anticancer agent should be switched to a treatment with another anticancer agent when a reduction of the therapeutic effect of the specific anticancer agent is observed. The anticancer agent can be the same as those described above.

In a specific embodiment, the method of the present invention may comprise:
(1) treating a sample with a protease; and
(2) measuring a fragment of the soluble c-MET which has the dipeptide unit GK at its C-terminus.

In (1), the sample is preferably the aforementioned liquid sample. When the liquid sample likely contains the cell expressing the full-length c-MET (in other words, membrane-bound c-MET), the method of the present invention may comprise removing such a cell as aforementioned.

When the sample is treated with the protease as aforementioned, the fragment of the soluble c-MET having the dipeptide unit GK at its C-terminus that is derived from the specific soluble c-MET which can be contained in the sample is produced. On the other hand, even if the different soluble c-MET and the soluble splicing variant are treated with the protease, no fragment of the soluble c-MET having the dipeptide unit GK at its C-terminus is produced. Thus, by measuring the fragment of the specific soluble c-MET having the dipeptide unit GK at its C-terminus, it is possible to evaluate the presence or absence and the amount of the specific soluble c-MET in the sample, and further it is also possible to qualitatively or quantitatively detect the c-MET amplification cell in the subject from whom the sample is derived.

The present invention also provides an affinity substance for the region specific for the soluble c-MET formed by the cleavage between the lysine residue (K) and the valine residue (V) in the partial region GKVI (SEQ ID NO:2) in the c-MET extracellular domain. The region specific for the soluble c-MET formed by the cleavage between the lysine residue (K) and the valine residue (V) in the partial region GKVI (SEQ ID NO:2) in the c-MET extracellular domain is as aforementioned, and preferably a peptide region consisting of an amino acid sequence of SEQ ID NO:10 or SEQ ID NO:11. The affinity substance is as aforementioned, preferably an antibody and more preferably a monoclonal antibody. The affinity substance may optionally be provided in the form of being labeled with the labeling substance as described above.

The present invention further provides a diagnostic agent for cancers comprising the affinity substance as described above. The cancer and the subject to be diagnosed are as described above.

The reagent of the present invention may be provided in a form of a kit comprising an additional component in addition to the affinity substance as described above. In this case, respective components contained in the kit may be provided in a mutually separated form, e.g., a form of being contained in a different vessel (e.g., tube). For example, when the affinity substance is not labeled with the labeling substance, such a kit may further comprise the labeling substance. Such a kit may also comprise the affinity substance for the fragment of the c-MET having the dipeptide unit VI at its N-terminus, which is formed by the above cleavage. Such a kit may further comprise the specific soluble c-MET as described above or a partial peptide thereof as a positive control.

When the reagent of the present invention is provided in the form of the kit, the kit may comprise an additional component depending on a type of the affinity substance as described above. For example, when the affinity substance is an antibody, the kit may further comprise a secondary antibody (e.g., anti-IgG antibody) and a reagent for detecting the secondary antibody.

When the reagent of the present invention is provided in the form of the kit, the kit may further comprise an instrument capable of collecting the sample such as the liquid sample. The instrument capable of collecting the sample is not particularly limited as long as the sample can be obtained from the subject, and examples of the instrument may include a blood collecting instrument such as a syringe.

### EXAMPLES

The present invention will be described in more detail with reference to following Examples, but the present invention is not limited thereto.

### Example 1: Secretion of soluble c-MET by ADAM17

A negative control si-RNA (si-N.C.) for the c-MET gene amplification lung cancer cell line NCI-H1993 and si-RNA (si-AD10 or si-AD17) targeting an ADAM10 gene or an ADAM17 gene were transfected. At a time point of 24 hours after the transfection, a medium was changed to a serum-free medium, and a culture supernatant after incubating the serum-free medium for 24 hours was collected. The culture supernatant filtrated through a filter was concentrated through an ultrafiltration membrane, subsequently developed on SDS-PAGE, and transferred onto a PVDF membrane. An effect of ADAM10 and ADAM17 knockdown on secretion of soluble c-MET was confirmed by a western blotting method using an antibody that recognized an extracellular domain (MET-α chain).

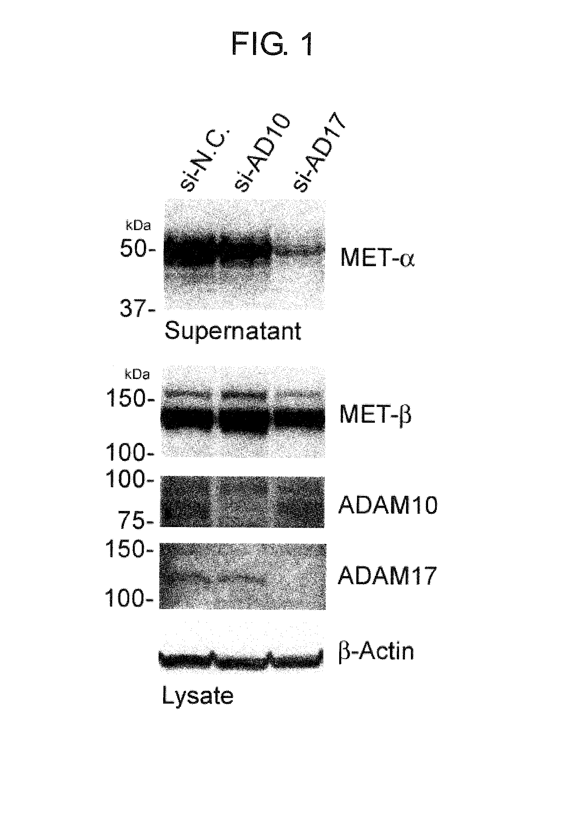

As a result, the secretion of c-MET was remarkably inhibited by the knockdown of ADAM17 whereas it was not inhibited by the knockdown of ADAM10 (FIG. 1). Thus, it has been shown that the specific soluble c-MET specific for a cancer cell, which is produced by cleavage of c-MET, is secreted mainly through a cleavage action of ADAM17.

### Example 2: Concentration of soluble c-MET by immunoprecipitation

The c-MET gene amplification lung cancer cell line NCI-H1993 at a sub-confluent state was incubated in the serum-free medium for 7 days, and subsequently a culture supernatant was collected. The culture supernatant filtrated through the filter was concentrated through the ultrafiltration membrane, and subsequently immunoprecipitated with an antibody that recognized an extracellular region of c-MET. The resulting immunoprecipitation complex was developed on non-reduced SDS-PAGE, and stained with a biosafety CBB staining solution.

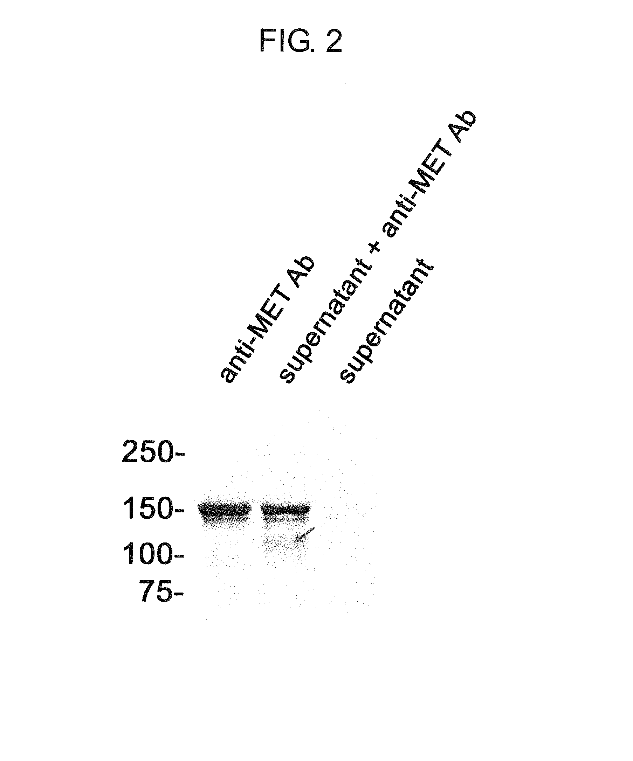

As a result, a band that was thought to correspond to the specific soluble c-MET (a fragment of a c-MET extracellular domain) produced by the cleavage of c-MET expressing in cancer cells, which was different from a band for the antibody of 150 kDa, was detected in a sample in which the culture supernatant had been mixed with the antibody (FIG. 2).

### Example 3: Identification of C-terminus candidate of soluble c-MET by LC-tandem mass spectrometry

A stained portion exhibiting a mobility corresponding to the soluble c-MET was cut out using a cutter, and digested with trypsin or chymotrypsin in the gel. The resulting digested product was analyzed by an LC-tandem mass apparatus (Thermo LTQ XL), and SEQUEST analysis was carried out by utilizing data set of a polypeptide consisting of a virtual sequence of the soluble c-MET as a reference sequence.

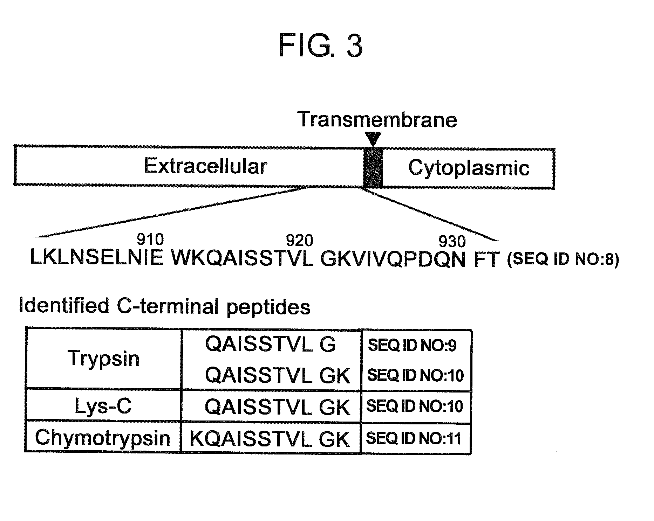

As a result, a peptide fragment having 921G and 922K at its C-terminus was detected in the product digested with trypsin, and a peptide fragment having 922K at its C-terminus was detected in the product digested with Lys-C (FIG. 3). A peptide fragment having 922K at its C-terminus was detected in the product digested with chymotrypsin. From sequence recognition profiles of trypsin, Lys-C and chymotrypsin, 921G was thought to be highly likely due to error digestion with trypsin or error recognition by the mass spectrometer. Thus, it has been shown that the amino acid residue at the C-terminus of the specific soluble c-MET secreted by the c-MET gene amplification cell is 922K as well as such a specific soluble c-MET is formed by the cleavage between the lysine residue (K) and the valine residue (V) in the partial region GKVI (SEQ ID NO:2) in the extracellular domain.

### Example 4: Tandem mass spectrum of C-terminus candidate peptide

A tandem mass spectrum derived from a C-terminal peptide contained in the product digested with trypsin or the product digested with chymotrypsin was identified based on information for m/z values of predicted precursor ions and product ions from all tandem mass spectrum data detected from soluble c-MET samples. The product ion having the predicted m/z value was imputed to each spectrum, and it was proven that the digested products contained a fragment digested with trypsin: QAISSTVLGK (SEQ ID NO:10) and a fragment digested with chymotrypsin: KQAISSTVLGK (SEQ ID NO:11).

Therefore, it has been shown that the soluble c-MET is formed by the cleavage between the lysine residue (K) and the valine residue (V) in the partial region GKVI (SEQ ID NO:2) in the extracellular domain in the c-MET gene amplification cell.

### Industrial applicability

The method of present invention is useful for examining the cancers, and the like.

## Claims

1. A method for detecting a c-MET gene amplification cell, comprising measuring a soluble c-MET in a sample utilizing a region specific for the soluble c-MET, wherein the soluble c-MET is formed by cleavage between the lysine residue (K) and the valine residue (V) in the partial region GKVI (SEQ ID NO:2) in a c-MET extracellular domain.

2. The method according to claim 1, which is a method of examining a cancer.

3. The method according to claim 1 or 2, wherein the sample is derived from a human.

4. The method according to any one of claims 1 to 3, wherein the sample is a liquid sample.

5. The method according to any one of claims 1 to 4, wherein the c-MET gene amplification cell is a lung cancer cell.

6. The method according to any one of claims 1 to 5, comprising:
(1) treating the sample with a protease; and
(2) measuring a fragment of the soluble c-MET which has the dipeptide unit GK at its C-terminus.

7. The method according to any one of claims 1 to 5, wherein the measurement of the soluble c-MET is carried out by detecting the fragment of the soluble c-MET which has the dipeptide unit GK at its C-terminus by mass spectrometry.

8. The method according to claim 6 or 7, wherein the soluble c-MET fragment is a peptide consisting of an amino acid sequence of QAISSTVLGK (SEQ ID NO:10) or KQAISSTVLGK (SEQ ID NO:11).

9. An affinity substance for a region specific for a soluble c-MET formed by cleavage between the lysine residue (K) and the valine residue (V) in the partial region GKVI (SEQ ID NO:2) in a c-MET extracellular domain.

10. The affinity substance according to claim 9, wherein the affinity substance is an antibody.

11. A diagnostic reagent for cancers, comprising an affinity substance for a region specific for a soluble c-MET formed by cleavage between the lysine residue (K) and the valine residue (V) in the partial region GKVI (SEQ ID NO:2) in a c-MET extracellular domain.


SEQUENCE LISTING

<110>  FUJIREBIO INC.

<120>  Method for detecting c-MET gene-amplifying cells

<130>  PRBA-26033

<150>  JP2013-027404
<151>  2013-02-15

<160>  11

<170>  PatentIn version 3.5

<210>  1
<211>  1390
<212>  PRT
<213>  Homo sapiens

<400>  1

Met Lys Ala Pro Ala Val Leu Ala Pro Gly Ile Leu Val Leu Leu Phe
1               5                   10                  15

Thr Leu Val Gln Arg Ser Asn Gly Glu Cys Lys Glu Ala Leu Ala Lys
            20                  25                  30

Ser Glu Met Asn Val Asn Met Lys Tyr Gln Leu Pro Asn Phe Thr Ala
        35                  40                  45

Glu Thr Pro Ile Gln Asn Val Ile Leu His Glu His His Ile Phe Leu
    50                  55                  60

Gly Ala Thr Asn Tyr Ile Tyr Val Leu Asn Glu Glu Asp Leu Gln Lys
65                  70                  75                  80

Val Ala Glu Tyr Lys Thr Gly Pro Val Leu Glu His Pro Asp Cys Phe
                85                  90                  95

Pro Cys Gln Asp Cys Ser Ser Lys Ala Asn Leu Ser Gly Gly Val Trp
            100                 105                 110

Lys Asp Asn Ile Asn Met Ala Leu Val Val Asp Thr Tyr Tyr Asp Asp
        115                 120                 125

Gln Leu Ile Ser Cys Gly Ser Val Asn Arg Gly Thr Cys Gln Arg His
    130                 135                 140

Val Phe Pro His Asn His Thr Ala Asp Ile Gln Ser Glu Val His Cys
145                 150                 155                 160

Ile Phe Ser Pro Gln Ile Glu Glu Pro Ser Gln Cys Pro Asp Cys Val

                165                 170                 175

Val Ser Ala Leu Gly Ala Lys Val Leu Ser Ser Val Lys Asp Arg Phe
            180                 185                 190

Ile Asn Phe Phe Val Gly Asn Thr Ile Asn Ser Ser Tyr Phe Pro Asp
        195                 200                 205

His Pro Leu His Ser Ile Ser Val Arg Arg Leu Lys Glu Thr Lys Asp
    210                 215                 220

Gly Phe Met Phe Leu Thr Asp Gln Ser Tyr Ile Asp Val Leu Pro Glu
225                 230                 235                 240

Phe Arg Asp Ser Tyr Pro Ile Lys Tyr Val His Ala Phe Glu Ser Asn
                245                 250                 255

Asn Phe Ile Tyr Phe Leu Thr Val Gln Arg Glu Thr Leu Asp Ala Gln
            260                 265                 270

Thr Phe His Thr Arg Ile Ile Arg Phe Cys Ser Ile Asn Ser Gly Leu
        275                 280                 285

His Ser Tyr Met Glu Met Pro Leu Glu Cys Ile Leu Thr Glu Lys Arg
    290                 295                 300

Lys Lys Arg Ser Thr Lys Lys Glu Val Phe Asn Ile Leu Gln Ala Ala
305                 310                 315                 320

Tyr Val Ser Lys Pro Gly Ala Gln Leu Ala Arg Gln Ile Gly Ala Ser
                325                 330                 335

Leu Asn Asp Asp Ile Leu Phe Gly Val Phe Ala Gln Ser Lys Pro Asp
            340                 345                 350

Ser Ala Glu Pro Met Asp Arg Ser Ala Met Cys Ala Phe Pro Ile Lys
        355                 360                 365

Tyr Val Asn Asp Phe Phe Asn Lys Ile Val Asn Lys Asn Asn Val Arg
    370                 375                 380

Cys Leu Gln His Phe Tyr Gly Pro Asn His Glu His Cys Phe Asn Arg
385                 390                 395                 400

Thr Leu Leu Arg Asn Ser Ser Gly Cys Glu Ala Arg Arg Asp Glu Tyr
                405                 410                 415

Arg Thr Glu Phe Thr Thr Ala Leu Gln Arg Val Asp Leu Phe Met Gly
            420                 425                 430

Gln Phe Ser Glu Val Leu Leu Thr Ser Ile Ser Thr Phe Ile Lys Gly
        435                 440                 445

Asp Leu Thr Ile Ala Asn Leu Gly Thr Ser Glu Gly Arg Phe Met Gln
    450                 455                 460

Val Val Val Ser Arg Ser Gly Pro Ser Thr Pro His Val Asn Phe Leu
465                 470                 475                 480

Leu Asp Ser His Pro Val Ser Pro Glu Val Ile Val Glu His Thr Leu
                485                 490                 495

Asn Gln Asn Gly Tyr Thr Leu Val Ile Thr Gly Lys Lys Ile Thr Lys
            500                 505                 510

Ile Pro Leu Asn Gly Leu Gly Cys Arg His Phe Gln Ser Cys Ser Gln
        515                 520                 525

Cys Leu Ser Ala Pro Pro Phe Val Gln Cys Gly Trp Cys His Asp Lys
    530                 535                 540

Cys Val Arg Ser Glu Glu Cys Leu Ser Gly Thr Trp Thr Gln Gln Ile
545                 550                 555                 560

Cys Leu Pro Ala Ile Tyr Lys Val Phe Pro Asn Ser Ala Pro Leu Glu
                565                 570                 575

Gly Gly Thr Arg Leu Thr Ile Cys Gly Trp Asp Phe Gly Phe Arg Arg
            580                 585                 590

Asn Asn Lys Phe Asp Leu Lys Lys Thr Arg Val Leu Leu Gly Asn Glu
        595                 600                 605

Ser Cys Thr Leu Thr Leu Ser Glu Ser Thr Met Asn Thr Leu Lys Cys
    610                 615                 620

Thr Val Gly Pro Ala Met Asn Lys His Phe Asn Met Ser Ile Ile Ile
625                 630                 635                 640

Ser Asn Gly His Gly Thr Thr Gln Tyr Ser Thr Phe Ser Tyr Val Asp
                645                 650                 655

Pro Val Ile Thr Ser Ile Ser Pro Lys Tyr Gly Pro Met Ala Gly Gly
            660                 665                 670

Thr Leu Leu Thr Leu Thr Gly Asn Tyr Leu Asn Ser Gly Asn Ser Arg
        675                 680                 685

His Ile Ser Ile Gly Gly Lys Thr Cys Thr Leu Lys Ser Val Ser Asn
    690                 695                 700

Ser Ile Leu Glu Cys Tyr Thr Pro Ala Gln Thr Ile Ser Thr Glu Phe
705                 710                 715                 720

Ala Val Lys Leu Lys Ile Asp Leu Ala Asn Arg Glu Thr Ser Ile Phe
                725                 730                 735

Ser Tyr Arg Glu Asp Pro Ile Val Tyr Glu Ile His Pro Thr Lys Ser
            740                 745                 750

Phe Ile Ser Gly Gly Ser Thr Ile Thr Gly Val Gly Lys Asn Leu Asn
        755                 760                 765

Ser Val Ser Val Pro Arg Met Val Ile Asn Val His Glu Ala Gly Arg
    770                 775                 780

Asn Phe Thr Val Ala Cys Gln His Arg Ser Asn Ser Glu Ile Ile Cys
785                 790                 795                 800

Cys Thr Thr Pro Ser Leu Gln Gln Leu Asn Leu Gln Leu Pro Leu Lys
                805                 810                 815

Thr Lys Ala Phe Phe Met Leu Asp Gly Ile Leu Ser Lys Tyr Phe Asp
            820                 825                 830

Leu Ile Tyr Val His Asn Pro Val Phe Lys Pro Phe Glu Lys Pro Val
        835                 840                 845

Met Ile Ser Met Gly Asn Glu Asn Val Leu Glu Ile Lys Gly Asn Asp
    850                 855                 860

Ile Asp Pro Glu Ala Val Lys Gly Glu Val Leu Lys Val Gly Asn Lys
865                 870                 875                 880

Ser Cys Glu Asn Ile His Leu His Ser Glu Ala Val Leu Cys Thr Val
                885                 890                 895

Pro Asn Asp Leu Leu Lys Leu Asn Ser Glu Leu Asn Ile Glu Trp Lys
            900                 905                 910

Gln Ala Ile Ser Ser Thr Val Leu Gly Lys Val Ile Val Gln Pro Asp
        915                 920                 925

Gln Asn Phe Thr Gly Leu Ile Ala Gly Val Val Ser Ile Ser Thr Ala
    930                 935                 940

Leu Leu Leu Leu Leu Gly Phe Phe Leu Trp Leu Lys Lys Arg Lys Gln
945                 950                 955                 960

Ile Lys Asp Leu Gly Ser Glu Leu Val Arg Tyr Asp Ala Arg Val His
                965                 970                 975

Thr Pro His Leu Asp Arg Leu Val Ser Ala Arg Ser Val Ser Pro Thr
            980                 985                 990

Thr Glu Met Val Ser Asn Glu Ser  Val Asp Tyr Arg Ala  Thr Phe Pro
        995                 1000                 1005

Glu Asp  Gln Phe Pro Asn Ser  Ser Gln Asn Gly Ser  Cys Arg Gln
    1010                 1015                 1020

Val Gln  Tyr Pro Leu Thr Asp  Met Ser Pro Ile Leu  Thr Ser Gly
    1025                 1030                 1035

Asp Ser  Asp Ile Ser Ser Pro  Leu Leu Gln Asn Thr  Val His Ile
    1040                 1045                 1050

Asp Leu  Ser Ala Leu Asn Pro  Glu Leu Val Gln Ala  Val Gln His
    1055                 1060                 1065

Val Val  Ile Gly Pro Ser Ser  Leu Ile Val His Phe  Asn Glu Val
    1070                 1075                 1080

Ile Gly  Arg Gly His Phe Gly  Cys Val Tyr His Gly  Thr Leu Leu
    1085                 1090                 1095

Asp Asn  Asp Gly Lys Lys Ile  His Cys Ala Val Lys  Ser Leu Asn
    1100                 1105                 1110

Arg Ile  Thr Asp Ile Gly Glu  Val Ser Gln Phe Leu  Thr Glu Gly
    1115                 1120                 1125

Ile Ile  Met Lys Asp Phe Ser  His Pro Asn Val Leu  Ser Leu Leu
    1130                 1135                 1140

Gly Ile  Cys Leu Arg Ser Glu  Gly Ser Pro Leu Val  Val Leu Pro
    1145                 1150                 1155

Tyr Met  Lys His Gly Asp Leu  Arg Asn Phe Ile Arg  Asn Glu Thr

1160                1165                1170

His Asn Pro Thr Val Lys Asp Leu Ile Gly Phe Gly Leu Gln Val
    1175                1180                1185

Ala Lys Gly Met Lys Tyr Leu Ala Ser Lys Lys Phe Val His Arg
    1190                1195                1200

Asp Leu Ala Ala Arg Asn Cys Met Leu Asp Glu Lys Phe Thr Val
    1205                1210                1215

Lys Val Ala Asp Phe Gly Leu Ala Arg Asp Met Tyr Asp Lys Glu
    1220                1225                1230

Tyr Tyr Ser Val His Asn Lys Thr Gly Ala Lys Leu Pro Val Lys
    1235                1240                1245

Trp Met Ala Leu Glu Ser Leu Gln Thr Gln Lys Phe Thr Thr Lys
    1250                1255                1260

Ser Asp Val Trp Ser Phe Gly Val Leu Leu Trp Glu Leu Met Thr
    1265                1270                1275

Arg Gly Ala Pro Pro Tyr Pro Asp Val Asn Thr Phe Asp Ile Thr
    1280                1285                1290

Val Tyr Leu Leu Gln Gly Arg Arg Leu Leu Gln Pro Glu Tyr Cys
    1295                1300                1305

Pro Asp Pro Leu Tyr Glu Val Met Leu Lys Cys Trp His Pro Lys
    1310                1315                1320

Ala Glu Met Arg Pro Ser Phe Ser Glu Leu Val Ser Arg Ile Ser
    1325                1330                1335

Ala Ile Phe Ser Thr Phe Ile Gly Glu His Tyr Val His Val Asn
    1340                1345                1350

Ala Thr Tyr Val Asn Val Lys Cys Val Ala Pro Tyr Pro Ser Leu
    1355                1360                1365

Leu Ser Ser Glu Asp Asn Ala Asp Asp Glu Val Asp Thr Arg Pro
    1370                1375                1380

Ala Ser Phe Trp Glu Thr Ser
    1385                1390

<210>  2
<211>  4
<212>  PRT
<213>  Artificial Sequence

<220>
<223>  Partial amino acid sequence from human c-MET

<400>  2

Gly Lys Val Ile
1


<210>  3
<211>  6
<212>  PRT
<213>  Artificial Sequence

<220>
<223>  Partial amino acid sequence from human c-MET

<400>  3

Leu Gly Lys Val Ile Val
1               5


<210>  4
<211>  8
<212>  PRT
<213>  Artificial Sequence

<220>
<223>  Partial amino acid sequence from human c-MET

<400>  4

Val Leu Gly Lys Val Ile Val Gln
1               5


<210>  5
<211>  10
<212>  PRT
<213>  Artificial Sequence

<220>
<223>  Partial amino acid sequence from human c-MET

<400>  5

Thr Val Leu Gly Lys Val Ile Val Gln Pro
1               5                   10


<210>  6
<211>  4
<212>  PRT
<213>  Artificial Sequence

<220>
<223>  Partial amino acid sequence from human c-MET

<400>  6

Val Leu Gly Lys
1



<210>  7
<211>  5
<212>  PRT
<213>  Artificial Sequence

<220>
<223>  Partial amino acid sequence from human c-MET

<400>  7

Thr Val Leu Gly Lys
1               5



<210>  8
<211>  32
<212>  PRT
<213>  Artificial Sequence

<220>
<223>  Partial amino acid sequence from human c-MET

<400>  8

Leu Lys Leu Asn Ser Glu Leu Asn Ile Glu Trp Lys Gln Ala Ile Ser
1               5                   10                  15


Ser Thr Val Leu Gly Lys Val Ile Val Gln Pro Asp Gln Asn Phe Thr
            20                  25                  30



<210>  9
<211>  9
<212>  PRT
<213>  Artificial Sequence

<220>
<223>  Partial amino acid sequence from human c-MET

<400>  9

Gln Ala Ile Ser Ser Thr Val Leu Gly
1               5



<210>  10
<211>  10
<212>  PRT
<213>  Artificial Sequence

<220>
<223>  Partial amino acid sequence from human c-MET

<400>  10

Gln Ala Ile Ser Ser Thr Val Leu Gly Lys
1               5                   10

<210>  11
<211>  11
<212>  PRT
<213>  Artificial Sequence

<220>
<223>  Partial amino acid sequence from human c-MET

<400>  11

Lys Gln Ala Ile Ser Ser Thr Val Leu Gly Lys
1               5                   10